# EUROPEAN PATENT APPLICATION

(11) **EP 3 692 845 A1**
(43) Date of publication of application: **12.08.2020**
(21) Application number: 19155908.7
(22) Date of filing: 07.02.2019
(51) Int. Cl.: A24F 47/00, A61M 15/00, B65D 83/68

(54) **SMOKING SUBSTITUTE APPARATUS**

(71) Applicant: Nerudia Ltd., Liverpool, Merseyside L24 9HP (GB)
(72) Inventor: The designation of the inventor has not yet been filed
(74) Representative: Mewburn Ellis LLP

(57) **Abstract**

A smoking substitute apparatus, comprising: a first compartment for receiving an aerosol former; a second compartment for receiving an property modifying agent configured to modify one or more properties of the aerosol former; a barrier configured to prevent fluid communication between the first and second compartments, wherein removing the barrier establishes said fluid communication and thereby allows the aerosol former and the property modifying agent to come into contact with each other.

## Description

### Field of the Invention

The present invention relates to a smoking substitute apparatus and, in particular, a smoking substitute apparatus able to deliver flavour to a user by adding a flavourant to an aerosol former.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Combustion of organic material such as tobacco is known to produce tar and other potentially harmful byproducts. There have been proposed various smoking substitute systems in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. There are a number of different categories of smoking substitute systems, each utilising a different smoking substitute approach.

One approach is the so-called "vaping" approach, in which a vaporisable liquid, typically referred to (and referred to herein) as "e-liquid", is heated by a heating device (referred to herein as an electronic cigarette or "e-cigarette" device) to produce an aerosol vapour which is inhaled by a user. The e-liquid typically includes a base liquid as well as nicotine and/or a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heater, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, e-liquid is heated by a heating device to produce an aerosol vapour which is inhaled by a user. Many e-cigarettes also deliver flavour to the user to enhance the experience. In such e-cigarettes, e-liquid is often sold as a flavoured product, e.g. a specific blend of flavour compounds is already homogeneously mixed with the e-liquid during the manufacturing process. As such, the user would have to purchase flavoured consumables available on the market with limited opportunities to personalise the vaping experience according to their preferences. Further, when blended in the e-liquid, flavour components may interact with other constituents in the e-liquid during storage. In addition, it may put additional strain on the supply chain for distributing a large variety of consumables each having different flavours.

There may be a need for improved design of smoking substitute systems, in particular in regards to the delivery of flavour to a user.

The present disclosure has been devised in the light of the above considerations.

### Summary of the Invention

At its most general, the present invention relates to a smoking substitute apparatus that allows an aerosol former to be stored separately from a property modifying agent, and only put into contact with each other prior to use. This may allow the user to flavour the aerosol former at the point of use. Therefore the smoking substitute apparatus may enable the property modifying agent to be kept separate to the aerosol former, it may minimise the interaction between the two during transport and storage. Further, it may allow the user to specify the quantity and type of property modifying agent to be added to the aerosol former, thus it may enable the user to create an aerosol former with a specific flavour and/or colour tailored to the user's needs.

According to a first aspect there is provided a smoking substitute apparatus, comprising:
a first compartment for receiving an aerosol former;
a second compartment for receiving an property modifying agent configured to modify one or more properties of the aerosol former;
a barrier configured to prevent fluid communication between the first and second compartments,
wherein removing the barrier establishes said fluid communication and thereby allows the aerosol former and the property modifying agent to come into contact with each other.

The aerosol former may be an e-liquid. The e-liquid may, for example, comprise a base liquid and nicotine. The base liquid may include propylene glycol and/or vegetable glycerine. The e-liquid may be flavourless. That is, the e-liquid may not contain any flavourant and may consist solely of a base liquid of propylene glycol and/or vegetable glycerine and nicotine.

The property modifying agent may comprise a flavourant. The flavourant may be provided in solid, gel or liquid form. It may include menthol, liquorice, chocolate, fruit flavour (including e.g. citrus, cherry etc.), vanilla, spice (e.g. ginger, cinnamon) and tobacco flavour. The flavourant may modify a flavour of the aerosol former upon contacting or mixing with the aerosol former.

The property modifying agent comprise be a colorant. The colorant may be provided in solid, gel or liquid form. The colorant may modify a colour of the aerosol former upon contacting or mixing with the aerosol former. Advantageously, when the colorant is provided with the flavourant in the property modifying agent, the colorant may provide visual indication to a user for indicating mixing progress. For example, the concentration of the flavourant may corresponds with a change in colour of the aerosol former.

The smoking substitute apparatus may allow the aerosol former and the property modifying agent to be kept in respective compartments and separate to each other during transportation and storage. The aerosol former and the property modifying agent may only come into contact once a user removes the barrier. This advantageously reduce the interaction between the aerosol former and the property modifying agent during transportation and storage. For example, the e-liquid may only be flavoured with flavourant prior to use.

Optionally, the first compartment is configured to be in fluid communication with the second compartment via a conduit. The first compartment may be adjacent to the second compartment or it may be spaced from the second compartment.

Optionally, the first compartment and the second compartment are separated by a partition wall. More specifically, the first compartment and second compartment may share the partition wall.

Optionally, the partition wall comprises an opening for effecting said fluid communication. As such, the opening provides a passage for the aerosol former in the first compartment to flow towards the second compartment, and for the flavour and/or colorant in the second compartment to enter the first compartment. Advantageously, the provision of an opening shortens the distance the aerosol former and the property modifying agent have to travel to come into contact with each other.

Optionally, the barrier is configured to close the opening prior to its removal. As such, the smoking substitute apparatus may be provided with the barrier blocking or sealing the opening. The barrier may be temporally or permanently be moved or removed from a sealing position that seals the opening, so as to establish said fluid communication.

Optionally, the barrier comprises a removable seal having a seal portion configured to seal the opening and a tab portion extending outwardly from the smoking substitute apparatus, whereby pulling the tab portion away from the smoking substitute apparatus detaches the seal portion from the opening and/or removes the removable seal from the smoking substitute apparatus. The seal portion may be formed together with the tab portion. The removable seal may seal the opening using induction sealing, adhesive sealing or any other sealing techniques known to the person skilled in the art. The tab portion may extend through the body of the smoking substitute apparatus such that the removable seal is accessible for a user. For example, the removable seal may extend through a slot extending from the opening towards an exterior wall of the aerosol forming device. By pulling on the tab portion, the seal portion may be forced to detach or break away from the opening and thereby establishes fluid communication between the first compartment and the second compartment. The removable seal may partially remain in the smoking substitute apparatus during use, or it may be completely withdrawn from the device.

Alternatively, the barrier comprises a valve for closing the opening, the valve is configured to be actuated by an actuator extending outwardly from the aerosol forming consumable; wherein actuating the actuator controls the flow of aerosol former and/or property modifying agent through said opening. The valve may be a check valve allowing the property modifying agent to flow from the second compartment towards the first compartment. Advantageously, the check valve may permit a one way flow passage for the property modifying agent, as such a user may control the dose of flavourant and/or colorant to be added to the aerosol former in the first compartment, whilst preventing an ingress of aerosol former into the second compartment. Alternatively, the valve may be a two way valve that permits free flow between the first and second compartments. Advantageously, the use of a two way valve may allow the use of valves that are simpler in construction.

The actuator may be a lever, a button, a toggle or other actuator that is accessible by the user. The valve may advantageously allow a user to control the flow of aerosol former and the property modifying agent between the first and second compartments. For example, a user may dispense a desired amount of the property modifying agent from the first compartment into the second compartment. This may advantageously allow the user to personalise the flavour and/or colour of the e-liquid by controlling the dosage of flavourant and/or colorant.

Alternatively, the barrier comprises a partition wall configured to separate the first compartment and the second compartment, wherein removing the partition wall merges the second compartment with the first compartment to form a combined compartment. For example, the partition wall may comprise a planar element movable along a pair of slots extending in between the first and second compartments. In use, a user may traverse the partition wall along the slot so as to remove the partition wall from the smoking substitute apparatus. This may enable the first and second compartments to merge into a single combined compartment, i.e. the aerosol former in the first compartment and the property modifying agent in the second compartment may mix in the combined compartment. Advantageously, such arrangement may allow the aerosol former and the property modifying agent to be mixed more promptly and efficiently in the combined compartment.

Optionally, the ratio of a volume of the first compartment to a volume of the second compartment is in the range of 1:1 to 9:1. More specifically, said ratio may be anyone of 1:1, 2:1, 3:1, 4:1, 5:1, 6:1, 7:1, 8:1 and 9:1, or any one of 50:50, 60:40, 70:30, 80:20 and 90:10. Providing the first and second compartments with predetermined ratios may advantageously allow the proportion of the aerosol former and property modifying agent to be specified, and the dosage of the property modifying agent may be made repeatable.

Optionally, the second compartment comprises a compartment inlet, wherein the second compartment is configured to receive the property modifying agent through the compartment inlet. Advantageously, this may allow the property modifying agent to be supplied separately to the aerosol forming device. More advantageously, a user may select a property modifying agent, or a mix of different property modifying agents, in a desired quantity, to be added to the aerosol former according to his/her preference. The compartment inlet may be closed by a trap door or a check valve so as to prevent the property modifying agent stored in the second compartment from escaping or leaking out from there.

Optionally, the compartment inlet at the second compartment is provided with a manually operated valve or flap, for example a hinged or sprung door which may be opened for receiving the property modifying agent and closed to seal the second compartment. This arrangement may be particularly suitable for property modifying agent that is provided as a solid puck, a block or a pellet of compressed powder or a substrate coated with property modifying agent. When the manually operated valve is closed, the second compartment may be filled with said property modifying agent.

Optionally, the compartment inlet is configured to engage with a dispenser outlet of a dispenser for dispensing the property modifying agent from the dispenser into the second compartment. Advantageously, this may allow the property modifying agent to be dispensed into the second compartment with greater efficiency and accuracy. The dispenser may comprise a bottle, a syringe, a pressurised canister, a squeezable tube or any other suitable dispenser. Optionally, the compartment inlet may only open when it is engaged with the dispenser outlet. For example, the trap door or check valve provided at the compartment inlet may only open upon engaging with a corresponding mechanism at the dispenser outlet. The compartment inlet may reseal upon disengaging the dispenser outlet therefrom. Advantageously, this may ensure the second compartment is assessable only when a corresponding dispenser is presented, as such it may prevent accidental discharge of the property modifying agent, as well as the tampering of the aerosol forming device.

Optionally, the property modifying agent may be in the form of any one or more of a solid, a gel, a liquid or a gas. For example, the flavourant and/or colourant may be provided as a powder, a pellet, a block or granules, and may readily dissolve in the aerosol former. The flavourant and/or colourant may be a gel that may readily dissolve in the aerosol former. The flavourant and/or colourant may be a liquid that may be miscible or immiscible with the aerosol former. The flavourant and/or colourant may be a gas that may be absorbed into the aerosol former.

According to a second aspect there is provided a dispenser for an aerosol forming device, comprising,
a storage for storing the property modifying agent; and
a dispenser outlet in communication with the storage;
wherein the dispenser outlet is configured to engage with a compartment inlet of the aerosol forming device so as to facilitate dispensing of the property modifying agent from the storage to a second compartment of the aerosol forming device.

The dispenser may comprise a bottle, a syringe, a pressurised canister, a squeezable tube or any other suitable dispenser. Advantageously, this may allow the property modifying agent to be dispensed into the second compartment with greater efficiency.

Optionally, the dispenser comprises a dosing mechanism for controlling the quantity of property modifying agent that is dispensed through the dispenser outlet. Optionally, the dispenser comprises an activator configured to activate the dispenser in proximity of the aerosol forming device.

For example, the property modifying agent, in a liquid form, may be added to the second compartment in the aerosol forming device by the dispenser, e.g. a bottle, syringe, pressurised canister or squeezable tube. To prevent spilling, the dispenser may comprise an activator, for example a docking mechanism or a key which only allows the liquid property modifying agent to flow when it is connected or engaged to, or in proximity of the aerosol forming device. The compartment inlet and the dispenser outlet may each be sealed by a check valve, e.g. diaphragm valve, ball valve, duckbill valve that only opens when the two are engaged. This may provide one way flow passage for the liquid property modifying agent to flow from the dispenser storage to the second compartment of the aerosol forming device, but not in the other direction. When the dispenser is disengaged or removed from the aerosol forming device, the valves on both the dispenser outlet and the compartment inlet may be closed.

The property modifying agent may be provided in a powder or a granular form, e.g. freeze dried granulate. Accordingly, the dispenser may be provided as a prefilled tube, a canister or a tank. Therefore, when the dispenser outlet is connected to the compartment inlet, it may allow a specified amount of powder to flow in to the second compartment. Such dispenser may have an activator, for example a docking mechanism or a key which only allows powder or granulate to flow when it is connected or engaged to, or in proximity of the aerosol forming device. For example, the dispenser may be activated by pushing on and/or twisting a portion of the dispenser to open a door or a check valve, e.g. diaphragm valve, a ball valve, a duckbill valve or other suitable valves. This may enable the powder to flow in to the second compartment. When the dispenser is disengaged or removed from the aerosol forming device, the valves on both the dispenser outlet and the compartment inlet may be closed.

The property modifying agent may be provided as a gas. Thereby the property modifying agent may be added by a dispenser in the form of a pressurised canister. More specifically, the dispenser outlet may dock with the compartment inlet through means of a mechanical connection e.g. a screw, a bayonet or a clip connection, so at to create a seal between the dispenser outlet and the compartment inlet. Advantageously, this may minimise or prevent the gaseous property modifying agent from escaping to the atmosphere. Upon engaging the dispenser outlet with the compartment inlet, the dispenser may disperse the gaseous property modifying agent in to the second compartment by means of a check valve, e.g. a diaphragm valve, a ball valve, a duckbill valve or other suitable valves, so as to provide one way flow passage for the gaseous property modifying agent to flow from the dispenser storage to the second compartment of the aerosol forming device, but not in the other direction. Upon removing the dispenser from the aerosol forming device, the valves on both the dispenser outlet and the compartment inlet may be closed.

Optionally, the activator of the dispenser is configured to recognise and identify markings or signals corresponding to an aerosol forming device, so as to confirm the presence of said device in its proximity. Upon confirming the presence of said device, the activator activates the dispenser, e.g. it actuates the valve at the dispenser outlet. Advantageously, such mechanism may prevent accidental discharge of property modifying agent, as well as prohibiting a user from tampering with the dispenser. As such that the dispenser only dispenses the property modifying agent in the presence of the aerosol forming device. For example, the activator may be an optical scanner configured to_scan and recognise an image (e.g. a barcode or QR code) printed on a surface the aerosol forming device to confirm its presence. Alternatively, the activator may be an RF scanner configured to read and recognise RF signals originated from an RFID at the aerosol forming device. Alternatively, the activator may respond to a magnetic field originated from a magnet at the aerosol forming device.

According to third aspect of there is provided a smoking substitute kit, comprising:
the smoking substitute apparatus;
the property modifying agent; and/or
the dispenser.

According to a fourth aspect there is provided a method of dispensing a property modifying agent to an aerosol former stored in a first compartment of an aerosol forming device, comprising:
providing the property modifying agent in a second compartment of the aerosol forming device;
removing a barrier that prevents fluid communication between the first compartment and the second compartment, thereby establishing said fluid communication to allow the aerosol former and the property modifying agent to come into contact with each other.

Optionally, said providing may comprise dispensing the property modifying agent in the second compartment.

Optionally, said providing may comprise dispensing the property modifying agent in the second compartment using a dispenser.

Optionally, the method may comprise moving the aerosol forming device to facilitate mixing of the aerosol former and the property modifying agent once they are put into contact with each other.

The smoking substitute apparatus may be in the form of a consumable. The consumable may be configured for engagement with a main body (i.e. so as to form a closed smoking substitute system). For example, the consumable may comprise components of the system that are disposable, and the main body may comprise non-disposable or non-consumable components (e.g. power supply, controller, sensor, etc.) that facilitate the delivery of aerosol by the consumable. In such an embodiment, the aerosol former (e.g. e-liquid) may be replenished by replacing a used consumable with an unused consumable.

Alternatively, the smoking substitute apparatus may be a non-consumable apparatus (e.g. that is in the form of an open smoking substitute system). In such embodiments an aerosol former (e.g. e-liquid) of the system may be replenished by re-filling e.g. a reservoir of the smoking substitute apparatus with the aerosol former (rather than replacing a consumable component of the apparatus).

In light of this, it should be appreciated that some of the features described herein as being part of the smoking substitute apparatus may alternatively form part of a main body for engagement with the smoking substitute apparatus (i.e. when the smoking substitute apparatus is in the form of a consumable).

Where the smoking substitute apparatus is in the form of a consumable, the main body and the consumable may be configured to be physically coupled together. For example, the consumable may be at least partially received in a recess of the main body, such that there is an interference fit between the main body and the consumable. Alternatively, the main body and the consumable may be physically coupled together by screwing one onto the other, or through a bayonet fitting.

Thus, the smoking substitute apparatus may comprise one or more engagement portions for engaging with a main body. In this way, one end of the smoking substitute apparatus may be coupled with the main body, whilst an opposing end of the smoking substitute apparatus may define a mouthpiece of the smoking substitute system.

The smoking substitute apparatus may comprise a reservoir configured to store an aerosol former, such as an e-liquid. The e-liquid may, for example, comprise a base liquid and e.g. nicotine. The base liquid may include propylene glycol and/or vegetable glycerine. The e-liquid may be flavourless. That is, the e-liquid may not contain any flavourant and may consist solely of a base liquid of propylene glycol and/or vegetable glycerine and nicotine.

The reservoir may be in the form of a tank. At least a portion of the tank may be translucent. For example, the tank may comprise a window to allow a user to visually assess the quantity of e-liquid in the tank. A housing of the smoking substitute apparatus may comprise a corresponding aperture (or slot) or window that may be aligned with a translucent portion (e.g. window) of the tank. The reservoir may be referred to as a "clearomizer" if it includes a window, or a "cartomizer" if it does not.

The smoking substitute apparatus may comprise a passage for fluid flow therethrough. The passage may extend through (at least a portion of) the smoking substitute apparatus, between openings that may define an inlet and an outlet of the passage. The outlet may be at a mouthpiece of the smoking substitute apparatus. In this respect, a user may draw fluid (e.g. air) into and through the passage by inhaling at the outlet (i.e. using the mouthpiece). The passage may be at least partially defined by the tank. The tank may substantially (or fully) define the passage. In this respect, the tank may surround the passage.

The smoking substitute apparatus may comprise an aerosol-generator. The aerosol generator may comprise a wick. The aerosol generator may further comprise a heater. The wick may comprise a porous material. A portion of the wick may be exposed to fluid flow in the passage. The wick may also comprise one or more portions in contact with liquid stored in the reservoir. For example, opposing ends of the wick may protrude into the reservoir and a central portion (between the ends) may extend across the passage so as to be exposed to fluid flow in the passage. Thus, fluid may be drawn (e.g. by capillary action) along the wick, from the reservoir to the exposed portion of the wick.

The heater may comprise a heating element, which may be in the form of a filament wound about the wick (e.g. the filament may extend helically about the wick). The filament may be wound about the exposed portion of the wick. The heating element may be electrically connected (or connectable) to a power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in fluid flowing through the passage. This vapour may subsequently cool to form an aerosol in the passage.

The smoking substitute apparatus (or main body engaged with the smoking substitute apparatus) may comprise a power source. The power source may be electrically connected (or connectable) to a heater of the smoking substitute apparatus (e.g. when engaged with the main body). The power source may be a battery (e.g. a rechargeable battery). A connector in the form of e.g. a USB port may be provided for recharging this battery.

When the smoking substitute apparatus is in the form of a consumable, the smoking substitute apparatus may comprise an electrical interface for interfacing with a corresponding electrical interface of the main body. One or both of the electrical interfaces may include one or more electrical contacts. Thus, when the main body is engaged with the consumable, the electrical interface may be configured to transfer electrical power from the power source to a heater of the consumable.

The electrical interface may also be used to identify the smoking substitute apparatus (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the smoking substitute apparatus is in the form of a consumable, the main body may comprise an interface, which may, for example, be in the form of an RFID reader, a barcode or QR code reader. This interface may be able to identify a characteristic (e.g. a type) of a consumable engaged with the main body. In this respect, the consumable may include any one or more of an RFID chip, a barcode or QR code, or memory within which is an identifier and which can be interrogated via the interface.

The smoking substitute apparatus or main body may comprise a controller, which may include a microprocessor. The controller may be configured to control the supply of power from the power source to the heater of the smoking substitute apparatus (e.g. via the electrical contacts). A memory may be provided and may be operatively connected to the controller. The memory may include non-volatile memory. The memory may include instructions which, when implemented, cause the controller to perform certain tasks or steps of a method.

The main body or smoking substitute apparatus may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. WiFi®, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater of the consumable in response to a puff detection by the sensor. The control may be in the form of activation of the heater in response to a detected puff. That is, the smoking substitute apparatus may be configured to be activated when a puff is detected by the puff sensor. When the smoking substitute apparatus is in the form of a consumable, the puff sensor may form part of the consumable or the main body.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
Figure 1A is a front view of a smoking substitute system, according to a first embodiment, in an engaged position;
Figure 1B is a front view of smoking substitute system of the first embodiment in a disengaged position;
Figure 1C is a section view of a smoking substitute apparatus of the smoking substitute system of Figures 1B and 1C;
Figure 2 is a front view of the smoking substitute apparatus according to a second embodiment;
Figure 3 is a front view of the smoking substitute apparatus according to a third embodiment; and
Figures 4A, 4B and 4C show sectional views of a dispenser according to a fourth embodiment.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

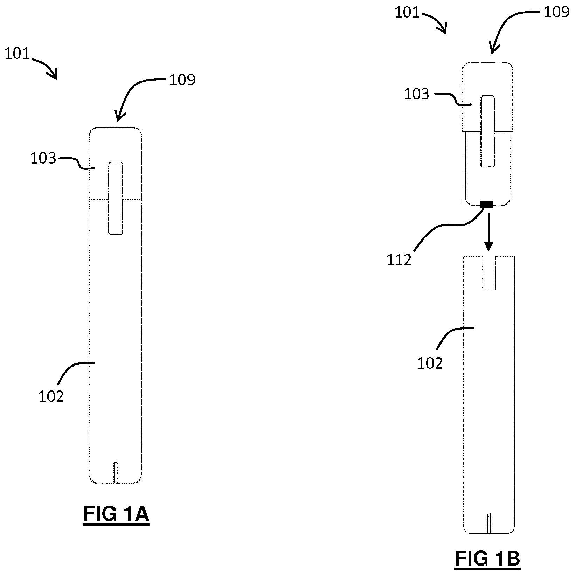

Figures 1A and 1B illustrate a smoking substitute system in the form of an e-cigarette system 101. The system 101 comprises an e-cigarette device defining a main body 102 of the system 101, and an smoking substitute apparatus in the form of an e-cigarette consumable (or "pod") 103. In the illustrated embodiment the consumable 103 (smoking substitute apparatus) is removable from the main body (e-cigarette device), so as to be a replaceable component of the system 101. In other words, the e-cigarette system 101 is a closed system.

As is apparent from Figures 1A and 1B, the consumable 103 is configured to engage the main body 102. Figure 1A shows the main body 102 and the consumable 103 in an engaged state, whilst Figure 1B shows the main body 102 and the consumable 103 in a disengaged state. When engaged, a portion of the consumable 103 is received in a cavity of the main body 102 and is retained in the engaged position by way of a snap-engagement mechanism. In other embodiments, the main body 102 and consumable 103 may be engaged by screwing one into (or onto) the other, through a bayonet fitting, or by way of an interference fit.

The system 101 is configured to vaporise an aerosol-former, which in the illustrated embodiment, is in the form of a nicotine-based e-liquid 104. The e-liquid 104 comprises nicotine and a base liquid including propylene glycol and/or vegetable glycerine. In the present embodiment, the e-liquid 104 is flavourless (and does not include any added flavourant). That is, if the e-liquid 104 were to be inhaled (i.e. in aerosol form) by a user, it would not have a particularly perceptible flavour or taste.

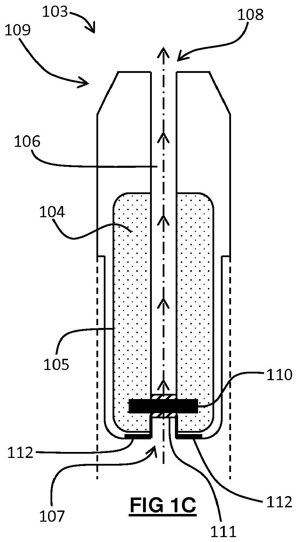

As is more apparent from Figure 1C, this e-liquid 104 is stored within a reservoir in the form of a tank 105 that forms part of the consumable 103. In the illustrated embodiment, the consumable 103 is a "single-use" consumable 103. That is, upon exhausting the e-liquid 104 in the tank 105, the intention is that the user disposes of the entire consumable 103. In other embodiments, the e-liquid (i.e. aerosol former) may be the only part of the system that is truly "single-use". That is, the tank may be refillable with e-liquid or the e-liquid may be stored in a non-consumable component of the system. For example, the e-liquid may be stored in a tank located in the main body or stored in another component that is itself not single-use (e.g. a refillable cartomizer).

The tank 105 surrounds, and thus defines a portion of, a passage 106 that extends between an inlet 107 and an outlet 108 at opposing ends of the consumable 103. In this respect, the passage comprises an upstream end at the end of the consumable 103 that engages with the main body 102, and a downstream end at an opposing end of the consumable 103 that comprises a mouthpiece 109 of the system 101. When the consumable 103 is engaged with the main body 102, a user can inhale (i.e. take a puff) via the mouthpiece 109 so as to draw air through the passage 106, and so as to form an airflow (indicated by arrows) in a direction from the inlet 107 to the outlet 108 of the passage 106. Although not illustrated, the passage 106 may be partially defined by a tube (e.g. a metal tube) extending through the consumable 103. The passage 106 is in fluid communication with a gap defined between the consumable 103 and the main body 102 (when engaged) such that air outside of the system 101 is drawn into the passage 106 (during an inhale).

The smoking substitute system 101 is configured to vaporise the e-liquid 104 for inhalation by a user. To provide this, the consumable 103 comprises a heater having of a porous wick 110 and a resistive heating element in the form of a heating filament 111 that is helically wound around a portion of the porous wick 110. The porous wick 110 extends across the passage 106 (i.e. transverse to a longitudinal axis of the passage106) and opposing ends of the wick 110 extend into the tank 105 (so as to be submerged in the e-liquid 104). In this way, e-liquid 104 contained in the tank 105 is conveyed from the opposing ends of the porous wick 110 to a central portion of the porous wick 110 so as to be exposed to the airflow in the passage 106 (i.e. caused by a user inhaling).

The helical filament 111 is wound about this exposed central portion of the porous wick 110 and is electrically connected to an electrical interface in the form of electrical contacts 112 mounted at the end of the consumable that is proximate the main body 102 (when engaged). When the consumable 103 is engaged with the main body 102, the electrical contacts 112 contact corresponding electrical contacts (not shown) of the main body 102. The main body electrical contacts are electrically connected to a power source (not shown) of the main body 102, such that (in the engaged position) the filament 111 is electrically connected to the power source. In this way, power can be supplied by the main body 102 to the filament 111 in order to heat the filament 111. This heat is transferred from the filament 111 to the porous wick 110 which causes e-liquid 104 conveyed by the porous wick 110 to increase in temperature to a point at which it vaporises. The vaporised e-liquid becomes entrained in the airflow and, between the vaporisation point at the filament 111 and the outlet 108 of the passage 106, condenses to form an aerosol. This aerosol is then inhaled, via the mouthpiece 109, by a user of the system 101.

The power source of the main body 102 may be in the form of a battery (e.g. a rechargeable battery). The main body 102 may comprise a connector in the form of e.g. a USB port for recharging this battery. The main body 102 may also comprise a controller that controls the supply of power from the power source to the main body electrical contacts (and thus to the filament 111). That, is the controller may be configured to control a voltage applied across the main body electrical contacts, and thus the voltage applied across the filament 111. In this way, the filament 111 may only be heated under certain conditions (e.g. during a puff and/or only when the system is in an active state). In this respect, the main body 102 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 102 and consumable 103 may comprise a further interface which may, for example, be in the form of an RFID reader, a barcode or QR code reader. This interface may be able to identify a characteristic (e.g. a type) of a consumable 103 engaged with the main body 102. In this respect, the consumable 103 may include any one or more of an RFID chip, a barcode or QR code, or memory within which is an identifier and which can be interrogated via the interface.

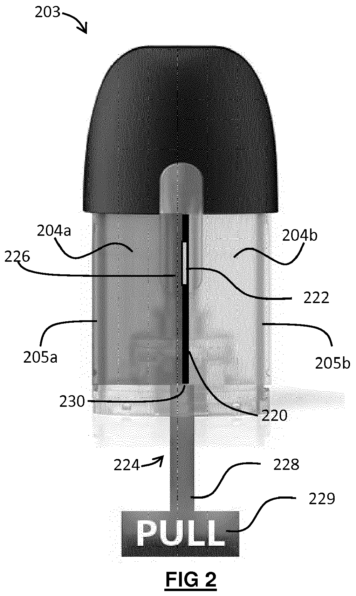

Figure 2 illustrates an aerosol forming device, or a consumable 203, according to an embodiment of the present invention. The consumable 203 comprises a tank 205 that is equally divided into a first compartment 205a and a second compartment 205b by a partition wall 220. In other words, the first compartment 205a and the second compartment 205b shares the partition wall 220. The partition wall 220 extends from a base of the consumable along the length of the first compartment 205a and second compartment 205b. In the illustrated embodiment the first compartment 205a and the second compartment 205b are of equal volume, e.g. 1:1. In other embodiments, the ratio of the volume of the first compartment to the volume of the second compartment may be in the range of 1:1 to 9:1.

In the illustrated embodiment, the first compartment 205a contains a supply of aerosol former, or e-liquid 204, and the second compartment 205b contains a property modifying agent, or an additive 204b. The aerosol former is in a liquid form. The aerosol former comprises nicotine and a base liquid, e.g. propylene glycol. The aerosol former does not contain any flavourant and therefore the aerosol former is not flavoured. The aerosol former in this embodiment does not contain any colourant. That is, the aerosol former is in the natural colour of the propylene glycol and the nicotine, and therefore it appears as a translucent or transparent liquid.

The property modifying agent 204b in the illustrated embodiment is a liquid property modifying agent, comprises a flavourant and a colourant respectively configured to modify the flavour and colour of the e-liquid 204 as stored in the first compartment 205a. In other embodiments, the property modifying agent may be a solid, a gel, a liquid or a gas, and may comprise only a flavourant without the presence of a colorant or may comprise a colorant without the presence of a flavourant.

The partition wall 220 as shown in Figure 2 comprises an opening 222. The opening 222 effects fluid communication between the first compartment 205a and the second compartment 205b. The opening 222 is sealed by a barrier or removable seal 224 prior to its removal. More specifically, a seal portion 226 of the removable seal 224 covers the opening 222 in a sealing position and thereby hermetically seals the opening 222. Therefore, in the sealing position, the seal portion 226 of the removable seal 224 prevents fluid communication between the first compartment 205a and the second compartment 205b. The removal seal 227 is configured to be removed from the sealing position and thereby detaches the seal portion 226 form the opening 222, and thereby establishes fluid communication between the first compartment 205a and second compartment 205b.

The removable seal 224 additionally comprises a tab portion 228 in connection with the seal portion 226. That is the seal portion 226 is formed together with the tab portion 228 to form the removable seal. Said tab portion 228 extends, along a slot in the consumable 203 and outwardly from the base of the consumable 203 through a check valve 230, such that at least a part of the tab portion 228 is accessible to a user. The tab portion 228 comprises an enlarged portion 229 external to the consumable 203 for a user to grip onto.

Prior to use or in use, the user may pull on the tab portion 228 away from the consumable 203 to detach the seal portion 226 from the opening 222. The removable seal 224 is then completely removed from the consumable 203 to be disposed of. Doing so establishes fluid communication between the first compartment 205a and second compartment 205b, thereby allowing the aerosol former 204a in the first compartment 205a to come into contact with the property modifying agent 204b in the second compartment 205b. More specifically, the property modifying agent 204b may diffuse into or mix with the aerosol former 204a to form a modified aerosol former once they come into contact with each other. Furthermore, a user may shake the consumable 203 to promote mixing of the aerosol former 204a and the property modifying agent 204b.

In the illustrated embodiment, the property modifying agent 204a comprises a mixture of flavourant and colorant. The colorant may serve as a visual indicator indicating the degree of homogeneity in the modified aerosol former. More specifically, the user may visually inspect the modified aerosol former through a translucent wall of the consumable 203. For example, a uniform distribution of colour in the modified aerosol former across the first and second compartment indicates that the flavourant and colorant are homogenously mixed with the aerosol former. On the other hand, localised fluctuation in colour signals uneven blending of the aerosol former 204a and the property modifying agent 204b and further mixing is required.

In another embodiment, the opening is closed by a valve that is actuated by an actuator extending outwardly from the consumable. The actuator is a lever that is mechanically linked to the valve to actuate the valve from a closed position which prevents fluid communication to an opened position which establishes fluid commutation between the first and second compartments.

In such embodiment, the valve is a check valve which allows the property modifying agent to flow from the second compartment towards the first compartment. This allows the flavourant and colorant to be dosed to the aerosol former in the first compartment, whilst preventing the aerosol former form entering the second compartment. In use, a user may actuate the actuator to control the flow of property modifying agent through said opening, and therefore dispense a desired amount of the property modifying agent from the first compartment into the second compartment. This advantageously allows the user to personalise the flavour and/or colour of the e-liquid by controlling the dosage of flavourant and/or colorant.

In another embodiment, the partition wall does not comprise an opening. Instead, the partition wall is removable for merging the second compartment with the first compartment to form a combined compartment. In this case, the partition wall comprises a planar element movable along a pair of slots extending in between the first and second compartments. In use, a user may pull the removable partition wall along the slot so as to remove said wall from the smoking substitute apparatus. The first and second compartments are then merge into a single combined compartment, i.e. the aerosol former in the first compartment and the property modifying agent in the second compartment may mix in the combined compartment.

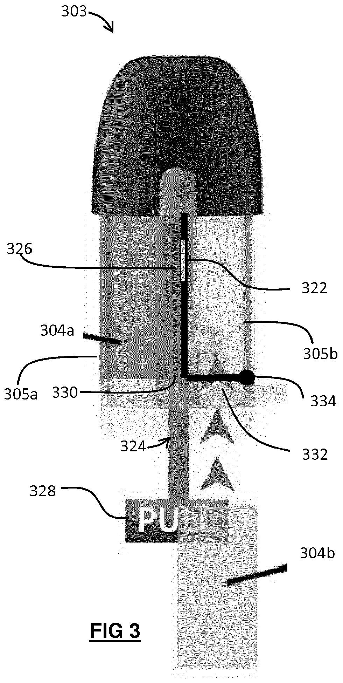

Figure 3 illustrates an aerosol forming device, or a consumable 303, according to another embodiment of the present invention. The consumable 303 is the similar in construction to the consumable 203 as shown in Figure 2. For example, the consumable 303 also comprises a partition wall 320 separating a first compartment 305a and second compartment 305b. A removable seal 324 is provided for sealing an opening 322 at the partition wall 320. Removing the removable seal 324 establishes fluid communication between the first compartment 305a and the second compartment 305b.

The consumable 303 differs to the consumable 203 of Figure 2 in that the consumable 303 additionally comprises a compartment inlet 332 at the base of the second compartment 305b. The compartment inlet 332 is closed by a cover 334 hingedly connected to the body of the consumable 303. The cover 334 is pivotable between a closed position that hermetically seals the second compartment 305b, and an open position that allows a property modifying agent 304b to be added to the second compartment 305b. More specifically, the consumable 303 is supplied with only an aerosol former 304a provided in the first compartment 305a. The second compartment 305b is empty having a void. Such arrangement allows the user to add a desired amount, and/or variety, of property modifying agent to the second compartment before it is mixed with the aerosol former. For example, a user may opt to add different flavourants (e.g. tobacco and blueberry flavour) into the second compartment 305b so as to create a personalised vaping experience.

The property modifying agent 304b in the illustrated example is a solid property modifying agent 304b comprising a mixture of flavourant and colourant. In use, a user may pivot the cover 334 from the close position towards the open position, before insert the solid property modifying agent 304b into the second compartment 305b. The user may then put the cover 334 into the close position to seal the second compartment 305b from the atmosphere.

Similar to the consumable 203 of Figure 2, upon removing the removable seal 324 from the consumable 303 fluid communication is established, via the opening 322, between the first compartment 305a and second compartment 305b. This allows the aerosol former 304a to flow through the opening 322 and contacts the solid property modifying agent 304b in the second compartment 305b. In the presence of aerosol former 304a, the solid property modifying agent 304b dissolves and thereby releases the flavourant and colourant into the aerosol former 304a.

The solid property modifying agent 304b may promptly dissolve in the aerosol former 304a, as such a modified aerosol former may be produced instantaneously. Alternatively, the solid property modifying agent 304b may dissolve gradually and therefore the concentration of flavourant and colourant in the modified aerosol former may vary with time. This allows the user to control the strength of flavour and colour in the generated aerosol according to his/her preference.

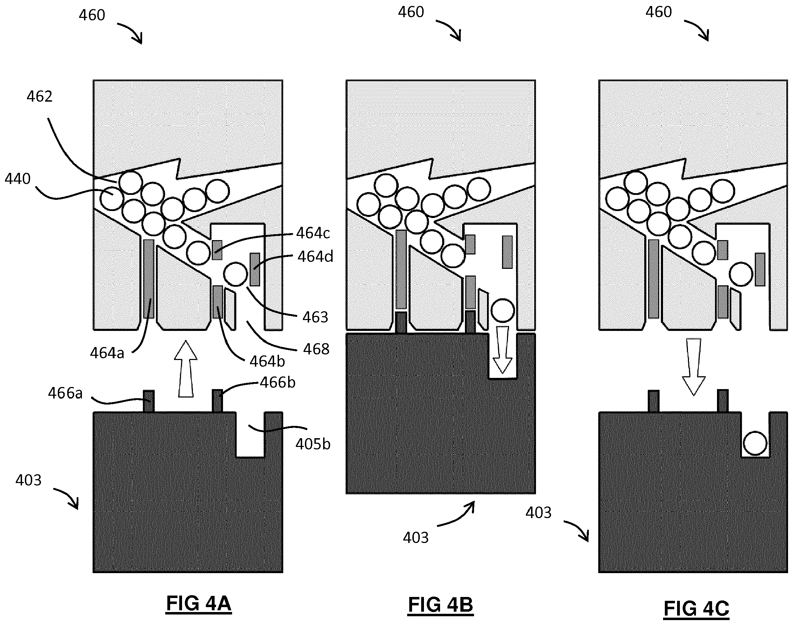

Figures 4a, 4b and 4c sequentially show a dispenser 460 dispensing a property modifying agent 440 to a second compartment 405b of a consumable 403. The property modifying agent 440 in this case is solid property modifying agent in the form of pellets, comprising flavourant and colourant. Figure 4a and Figure 4c show the dispenser 460 being separated from the consumable 403 and Figure 4b shows the dispenser 460 is engaged with the consumable 403. The dispenser 460 comprises a storage 462 for storing a supply of pellets 440 and a dispensing chamber 463 for storing a predetermined number of pellets to be dispensed. For example, the dispensing chamber 463 is sized to receive the required number of pellets to be dispensed, in this case a single pellet. However the dispensing chamber 463 may be sized to receive a plurality of pellets such that said plurality of pellets may be dispensed at once. When the dispenser is separated from the consumable, as shown in Figure 4a, a dispensing element 464d prevents the pellet from being dispensed out of the dispensing chamber 463.

The dispenser comprises a dispensing mechanism, which is formed of dispensing elements 464a, 464b, 464c and 464d, and is configured to be activated by a corresponding activating mechanism 466a and 466b at or near a compartment inlet of the consumable 403. More specifically, the activating mechanism 466a and 466b are protrusions that, when the dispenser 460 is engaged with the consumable 403 as shown in Figure 4B, push onto the dispensing element 464a and 464b and thereby urges the dispensing mechanism towards an upward direction. This causes the dispensing element 464d to move and thereby allows the pellet 440 in the dispensing chamber 463 to dispense through a dispenser outlet 468 into the second compartment 405b. Furthermore, when the dispensing mechanism is activated as shown in Figure 4B, a slot formed between the dispensing element 464b and 464c also moves in line with a pellet 440 stored in the storage 462 and thereby allowing the pellet 440 in the storage 462 to drop into the dispensing chamber 463. That is, activating the dispensing mechanism causes a pellet to be dispensed out of the dispensing chamber 463, as well as allowing a pellet from the storage 462 to replenish the dispensing chamber.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A smoking substitute apparatus, comprising:
a first compartment for receiving an aerosol former;
a second compartment for receiving an property modifying agent configured to modify one or more properties of the aerosol former;
a barrier configured to prevent fluid communication between the first and second compartments,
wherein removing the barrier establishes said fluid communication and thereby allows the aerosol former and the property modifying agent to come into contact with each other.

2. The smoking substitute apparatus of claim 1, wherein the first compartment and the second compartment are separated by a partition wall, said partition wall comprises an opening for effecting said fluid communication, wherein the barrier is configured to close the opening prior to its removal.

3. The smoking substitute apparatus of claim 2, wherein the barrier comprises a removable seal having a seal portion configured to seal the opening and a tab portion extending outwardly from the smoking substitute apparatus, whereby pulling the tab portion away from the smoking substitute apparatus detaches the seal portion from the opening and/or removes the removable seal from the smoking substitute apparatus.

4. The smoking substitute apparatus of claim 2, wherein the barrier comprises a valve for closing the opening, the valve is configured to be actuated by an actuator extending outwardly from the smoking substitute apparatus; wherein actuating the actuator controls the flow of aerosol former and/or property modifying agent through said opening.

5. The smoking substitute apparatus of claim 1, wherein the barrier comprises a partition wall configured to separate the first compartment and the second compartment, wherein removing the partition wall merges the second compartment with the first compartment to form a combined compartment.

6. The smoking substitute apparatus of any one of the preceding claims, wherein the ratio of a volume of the first compartment to a volume of the second compartment is in the range of 1:1 to 9:1.

7. The smoking substitute apparatus of any one of the preceding claims, wherein the second compartment comprises a compartment inlet, wherein the second compartment is configured to receive the property modifying agent through the compartment inlet.

8. The smoking substitute apparatus of claim 7, wherein the compartment inlet is configured to engage with a dispenser outlet of a dispenser for dispensing the property modifying agent from the dispenser into the second compartment.

9. The smoking substitute apparatus of any one of the preceding claims, wherein the property modifying agent is in the form of any one of more of a solid, a gel, a liquid and a gas.

10. The smoking substitute apparatus of any one of the preceding claims, wherein the property modifying agent comprises a flavourant and/or a colourant.

11. A dispenser for an aerosol forming device, comprising:
a storage for storing the property modifying agent; and
a dispenser outlet in communication with the storage;
wherein the dispenser outlet is configured to engage with a compartment inlet of a smoking substitute apparatus so as to facilitate dispensing of the property modifying agent from the storage to a second compartment of the smoking substitute apparatus.

12. The dispenser of claim 11, wherein the dispenser comprises an activator configured to activate the dispenser in proximity of the aerosol forming device.

13. A smoking substitute kit, comprising:
the smoking substitute apparatus of any one of the claims 1 to 10;
the property modifying agent of any one of the claims 1 to 10; and/or
the dispenser of any one of the claim 11 or claim 12.

14. A method of dispensing a property modifying agent to an aerosol former stored in a first compartment of an smoking substitute apparatus, comprising:
providing the property modifying agent in a second compartment of the smoking substitute apparatus;
removing a barrier that prevents fluid communication between the first compartment and the second compartment, thereby establishing said fluid communication to allow the aerosol former and the property modifying agent to come into contact with each other.

15. The method of claim 14, wherein said providing comprise dispensing the property modifying agent in the second compartment using a dispenser.
